(19) Europäisches Patentamt
European Patent Office
Office européen des brevets

(11) **EP 2 827 130 A1**

(12) **EUROPEAN PATENT APPLICATION**

(43) Date of publication:
**21.01.2015 Bulletin 2015/04**

(51) Int Cl.:
**G01N 21/64** (2006.01)     **G01N 33/543** (2006.01)
**G01N 33/551** (2006.01)

(21) Application number: **13176536.4**

(22) Date of filing: **15.07.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **F. Hoffmann-La Roche AG
4070 Basel (CH)**

(72) Inventor: **The designation of the inventor has not
yet been filed**

(74) Representative: **Bohest AG
Holbeinstrasse 36-38
4051 Basel (CH)**

(54) **Device for use in the detection of binding affinities**

(57)     Device (1) for use in the detection of binding affinities comprising a substrate (2) with a planar surface (21) having arranged thereon a plurality of binding sites (31) capable of binding with a target molecule (32), wherein the binding sites (31) are arranged on the planar surface (21) along a plurality curved lines (4) which are spaced from one another by a distance to cause an incident beam of coherent light of a predetermined wavelength generated at a beam generation location to be diffracted at the binding sites (31) with the bound target molecule (32) in a manner such that diffracted portions of the incident beam of coherent light interfere at a detection location with a difference in optical path length which is a multiple integer of the predetermined wavelength of the coherent light to provide at the detection location an signal representative of the binding affinity of the binding sites (31) and the target molecule (32), and further comprising a beam stop (72) which is arranged to prevent non-diffracted portions of the incident beam of coherent light at the detection location.

Fig. 1

EP 2 827 130 A1

**Description**

The present invention relates to a device for use in the detection of binding affinities between a binding site and a target molecule and to a method for detecting binding affinities.

Optical biosensors are devices which enable the detection of binding affinities. The binding affinity refers to the strength of the molecular interaction (e.g. a high affinity binding results from greater intermolecular force between the binding site and the target molecule). A typical field of application for such optical biosensor is the detection of binding affinities between receptor molecules (as binding sites) and a predetermined target molecule without limitation to any specific chemical, biological of pharmaceutical substance of interest. This detection can be carried out in a sandwich-assay in which the target molecule has bound to the binding site and to a labelled complementary binder molecule. The light emanating from the label is measured to detect the binding affinities. Alternatively, the capability of the target molecule to bind to the receptor molecule can be detected in a label-free manner.

In Surface Plasmon Resonance Spectroscopy (SPR) an optical biosensor enables the label-free detection by spectroscopically measuring a resonance shift in an absorption spectrum. The spectroscopic signal characteristically changes if a target molecule has bound to the attached receptor molecules and this change is representative of the binding affinity.

A state of the art SPR-device comprises a transparent substrate comprising at one side thereof a metal layer (e.g. a thin gold layer). A prism is arranged at the side of the substrate opposite to the side at which the metal layer is arranged. Receptor molecules as binding sites are immobilized on the metal layer. Target molecules are applied to the receptor molecules for the detection of the binding affinity between the receptor molecules and the target molecules.

During use of such device, an incident beam of light is directed via the prism through the transparent substrate onto the metal layer. The incident beam of light electromagnetically couples with a surface plasmon. The surface plasmon is a coherent electron oscillation that occurs at the interface of the metal and the medium above the outer surface of the metal and which propagates along the metal layer. Characteristically, the plasmon changes if attached receptor molecules have bound to applied target molecules. In terms of physics, the resonance frequency of the propagating surface plasmon changes characteristically in relation to occurring binding events between the receptor molecules and the target molecule. The reflected portion of the incident light is spectroscopically analysed by measuring changes in an angular absorption spectrum which provides a signal representative of occurring binding events between the receptor molecules and the target molecules.

A disadvantage associated with the above described SPR-device is the need for a metal layer to which the target molecule may bind unspecifically. Another disadvantage is that the sensitivity is limited by changes in the refractive index which accumulate over the propagation path of the plasmon.

It is an object of the present invention to provide a device for use in the detection of binding affinities between binding sites and a target molecule which overcomes or at least greatly reduces the disadvantages associated with prior art devices.

In accordance with the invention, this object is achieved by a device for use in the detection of binding affinities. The device comprises a substrate with a planar surface having arranged thereon a plurality of binding sites capable of binding with a target molecule. The binding sites are arranged on the planar surface along a plurality of adjacently arranged curved lines which are spaced from one another by a distance to cause an incident beam of coherent light of a predetermined wavelength generated at a beam generation location to be diffracted at the binding sites with the bound target molecule in a manner such that diffracted portions of the incident beam of coherent light interfere at a detection location with a difference in optical path length which is a multiple integer of the predetermined wavelength of the coherent light to provide at the detection location a signal representative of the binding affinity of the binding sites and the target molecule. The device further comprises a beam stop which is arranged to prevent non-diffracted portions of the incident beam of coherent light at the detection location.

For the detection of binding affinities, the binding sites are arranged along the plurality of curved lines and the target molecule is applied to the binding sites. In general, "binding sites" are locations on the planar surface to which a target molecule may bind (or binds in case of binding affinity). The detection of binding affinities according to the invention is neither limited to any specific types of target molecules nor to any type of binding sites, but rather the binding characteristics of molecules, proteins, DNA etc. as target molecules can be analysed with respect to any suitable type of binding sites on the planar surface. The diffracted "portion" refers to that not the entire incident beam of coherent light is diffracted so that a portion (in fact the main portion) continues to propagate in the direction of the incident beam. The incident beam of coherent light of a predetermined wavelength is generated in the beam generation location which may be a laser light source, and propagates to impinge on the curved lines so that a portion thereof is diffracted and propagates towards the detection location. The diffracted portions of the incident beam of coherent light interfere at the detection location (e.g. sensing surface of an optical CCD or CMOS detector) to provide a maximum signal. The signal in the detection location is compared with a reference which is for example the signal of the light diffracted at the binding sites only or with a (known) reference signal which is representative of a specific binding affinity between the binding sites and the target molecule. Alternatively, a time resolved measurement can be carried out to measure the signal representative of the binding affinity which is to be compared to a past signal representative of the binding affinity which has been detected before a predetermined time-interval. The propagation of the non-diffracted portion of the incident beam of coherent light into the detection location is prevented if the non-diffracted portion is completely masked out or if the intensity of the non-diffracted portion is reduced. The reduction needs to be such that the signal in the detection location is not adversely affected by the non-diffracted light so that an interpretation of the signal is possible. Technically, the term "non-diffracted" may be interpreted to include all portions of the incident beam of coherent light which are different from the diffracted portion. Particularly, the non-diffracted portion may be a reflected portion (reflected at a surface inside or outside the substrate), a refracted portion (refracted at an interface between the substrate and the surrounding medium) or a portion (part) of the incident beam which directly propagates into the detection location.

Since the diffracted light is of low intensity compared with the intensity of non-diffracted light, e.g. which is reflected or refracted at the substrate, the propagation of the non-diffracted portion of the incident beam of light into the detection location needs to be prevented by the beam stop. Advantageously, the detected intensity signal originating from the target molecules bound to the binding sites arranged along these lines scales with the square of the quantity of bound target molecules compared with the signal originating from unspecifically bound (randomly arranged and not along the curved lines arranged) target molecules for which the detected intensity signal scales with the quantity of the target molecules. In principle, this renders obsolete (or at least reduces the need for) washing away unspecifically bound target molecules from the planar surface prior to detection of the signal originating from specific binding of target molecules to binding sites on the planar surface.

The diffracted portions from separate locations of the same line as well as from locations from different lines contribute to the maximum at the detection location as long as the requirement that the difference in optical path length at the detection location is a multiple integer of the predetermined wavelength is fulfilled for the different portions. This can be achieved by lines which are arranged as a phase grating that forms a diffractive lens which focusses the diffracted portions into the detection location in a diffracting manner so that the diffracted portions interfere in the focal location (detection location), i.e. with curved lines having a graded distance. The intensity of the signal increases inter alia with the number of lines (assuming a constant density (number per surface area) of diffraction centers (molecules) along the curved line) at which a portion of the incident beam of coherent light diffracts the incident beam of coherent light at the binding sites and the target molecules bound to the binding sites. The distance between adjacent lines varies and ranges in a particular example from 260 nm to 680 nm. Lines within the meaning of the present invention are ideal lines which define the locations at the planar surface at which the binding sites are arranged. Deviations (in particular random deviations) of the arrangement of the diffraction centers (molecules) from the ideal lines, i.e. variations of the distance of the diffraction centers from the ideal lines, do not deteriorate the signal at the detection location, as long as the majority of such deviations are smaller than a quarter of the distance between adjacent lines. The optical path length is the product of the predetermined wavelength of the coherent light and the refractive index of the material through which the coherent light propagates, e.g. air, sample solution (aqueous solution $\approx 1.33$) or a liquid immersion ($n_{immersion} \approx n_{substrate}$) or the substrate ($n_{glass}$ = 1.521), respectively. In best case, the diffracted portions interfere in the detection location having a spatially distributed intensity profile of an Airy disk (i.e. the focused spot of light that an "ideal" lens with a circular aperture produces limited by the diffraction of the light) having a diameter given by Abbe's formula ($D = \lambda / (2NA)$; wherein D is the diameter of the (circular) area covered by the curved lines; NA is the numerical aperture which is defined technically analog to an aperture of a microscope).

Preferably, the substrate comprises a material which is transparent to light of the predetermined wavelength of the coherent light so as to allow for propagation of the incident beam of coherent light and of the diffracted portion of coherent light through the substrate. This allows an advantageous use of the device in which the incident beam of light is directed through the substrate to the curved lines and is diffracted therefrom again through the substrate to the detection location. In a particular example, the complete substrate is of the transparent material.

Advantageously, the beam stop comprises a non-transparent section which is arranged on or within the substrate in a manner to prevent non-diffracted portions (reflected portions) of the incident beam of coherent light at the detection location. The non-transparent section can be a section of the substrate of a light absorbing material or a separate element arranged in or on the substrate.

According to one aspect, the beam stop comprises an anti-reflective section arranged at the planar surface of the substrate. This allows for preventing the non-diffracted portions (reflected portions) of the incident beam of coherent light to propagate into and to impinge at the detection location. In one particular example, the anti-reflective layer is an optical coating of the planar surface having of a thickness and material so as to be capable of reducing or preventing non-diffracted portions (reflected portions) of light.

In a further embodiment of the invention, the beam stop comprises a deflector body arranged on or within the substrate. The deflector body has a curved outer surface which is capable of scattering off the coherent light in a manner such that the non-diffracted portion (reflected portion) of the incident beam of coherent light impinging at the detection location is dissipated. Dissipation means the reduction of the intensity of the non-diffracted portion (reflected portion) impinging at the detection location to an extent such that the diffracted portion of the incident beam of coherent light can be detected to provide a signal representative of the binding affinity. The deflector body could be embodied as a metallic sphere and can be arranged in the substrate adjacent to the planar surface. The metallic sphere has a curved outer metal surface which is capable of deflecting the incident beam of coherent light over a wide angle in a dissipating manner so as to propagate in a variety of directions with a reduced intensity compared with the intensity when the non-diffracted portion (reflected portion) propagates into only one direction.

According to one aspect, the beam generation location and the detection location are arranged on (or close to) an opposite outer surface of the substrate opposite to the planar surface. The beam generation location and the detection location are arranged on (or close to) the opposite outer surface in a common plane parallel to the planar surface. The beam generation location can be arranged at the opposite outer surface by attaching a laser light source thereto which generates a laser light beam propagating as incident beam of coherent light. The detection location can be arranged at the opposite outer surface by attaching a CCD or CMOS detector at the opposite outer surface of which a detection surface is the beam generation location.

According to a further aspect, the device comprises a carrier having the beam generation location and the detection location arranged thereon. The carrier is arranged with respect to the substrate such that the beam generation location and the detection location are arranged in a common plane parallel to the planar surface. Depending on the size and the geometry of the plurality of curved lines, it may be of advantage to arrange the beam generation location and the detection location at the carrier. This allows varying the distance in between of the detection location and the lines arranged at the substrate. This is particularly advantageous in case of differences in the dimensions of the device, e.g. dimensional changes caused by manufacturing tolerances.

According to one aspect, each line of the plurality of curved lines is arranged on the planar surface such that the integrated optical path length of the coherent light from the beam generation location to the curved line and the optical path length of the diffracted portion of the coherent light from the respective curved line to the detection location is constant. For the beam generation location and the detection location which are arranged separate in a plane parallel to the planar surface, the constant distance results in curved lines of an elliptic geometry. In a particular example, the curved lines are arranged in an elliptical manner so as to be geometrically defined in an x,y-plane at the planar surface by the following equation.

$$y_j{}^2 = \left(\frac{(j_0+j)\,\lambda}{2\,n_s}\right)^2 + \left(\frac{2\,n_s\;\xi\;x_j}{(j_0+j)\,\lambda}\right)^2 - \left(f^2+\xi^2+x_j{}^2\right)$$

wherein

$j_0$ ,j is an integer,
$\lambda$ is the predetermined vacuum wavelength of the coherent light,
$n_s$ is the refractive index of the substrate,
$\xi$ is half distance between the beam generation location and the detection location and
f is a number which corresponds to the distance between the center of the curved lines and the detection location.

According to one aspect, adjacent curved lines of the plurality of curved lines are arranged at a distance such that the integrated optical path length of the coherent light from the beam generation location to different curved lines of the plurality of curved lines and of the diffracted portion of the coherent light from the respective different curved lines to the detection location has a difference which is a multiple integer of the predetermined wavelength of the coherent light. This causes the light from the beam generation location to constructively interfere in the detection location to provide a maximum signal.

According to one aspect, the detection location is a sensing surface of a CCD or CMOS detector having a plurality of pixels arranged on the sensing surface in a grid-like manner. Each pixel has a size so as to be capable of detecting (or accommodating thereon) less than half of the spatially distributed intensity of the interfering diffracted portions at the detection location in an individual pixel (or on a predetermined number of adjacent pixels). The sensing surface comprises a two dimensional grid of pixels which allows for detecting the intensity of the interfering diffracted portions of the incident beam of coherent light. In a specific example, the pixel size is smaller than 1 $\mu m^2$ (square micrometer) to allow for detecting the focus in a single pixel without background signal.

According to one aspect, the beam generation location is a point light source capable of generating a divergent incident beam of coherent light. The point light source may be a laser beam propagating through a small aperture to cause an emanating spherical wave.

According to an aspect, an outer layer is arranged at the planar surface, the outer layer may be an outer compact layer or an outer porous layer. The outer porous layer may comprise a material having porosity such that only (or preferentially) target molecules protrude through the outer porous layer. The outer compact layer or outer porous layer can be arranged directly on the planar surface or at a distance so as to form a channel through which a fluid (e.g. a fluid capable of flowing the target molecules or a fluid capable of flowing complementary binder molecules which may be capable of binding to the target molecule and which carry a scattering enhancer) may flow.

According to a variant of the invention, a method for detecting binding affinities is provided, the method comprising the steps of:

- providing a device as described herein, in particular according to anyone of the device claims; thereafter
- applying a plurality of target molecules to the binding sites; thereafter
- at a beam generation location, generating an incident beam of coherent light to be diffracted at the binding sites with the target molecule bound thereto in a manner such that diffracted portions of the incident beam of coherent light interfere at a detection location with a difference in optical path length which is a multiple integer of the predetermined wavelength of the incident beam of coherent light to provide a signal representative of the binding sites with the target molecule bound thereto at the detection location; thereafter
- at the detection location, measuring the signal representative of the binding sites with the target molecule bound thereto; and thereafter
- detecting the binding affinity of the binding sites to the target molecule by comparing the signal representative of the binding sites with the target molecule bound thereto with a known signal representative of the binding sites only.

Preferably, the method before applying the plurality of target molecules to the binding sites comprises the steps of:

- at the beam generation location, generating the incident beam of coherent light which is to be diffracted at the binding sites only in a manner such that diffracted portions of the incident beam of coherent light interfere at the detection location with a difference in optical path length which is a multiple integer of the predetermined wavelength of the incident beam of coherent light to provide a signal representative of the binding sites only at the detection location; and before or thereafter
- at the detection location, measuring the signal representative of the binding sites only to provide the reference signal.

Preferably, the method further comprises the step of applying a plurality of complementary binder molecules to the binding sites, each complementary binder molecule being capable of binding to the target molecule bound to the binding site, and the complementary binder molecules comprising a scattering enhancer. In a particular example, the scattering enhancer comprises the material gold (gold nano-particle) and has a size of several nanometer, in particular in the range of 10 nm to 150 nm. A change in size of the scattering enhancer may be capable of changing the sensitivity of the detection of binding affinities. In one example, multiple complementary binder molecules can bind to a single scattering enhancer to allow for multiple bond interactions showing a combined strength (avidity).

Further advantageous aspects of the invention become apparent from the following description of embodiments of the invention with reference to the accompanying drawings in which:

Fig. 1  shows a plan view from above of a device according to an embodiment of the invention having a plurality of elliptically curved lines and a deflector body as beam stop;

Fig. 2  shows a side view on the device of Fig. 1 and illustrates the optical path's of the incident beam of light as well as the diffracted and reflected portions thereof;

Fig. 3  shows the side view of the device of Fig. 1 having a carrier, wherein the incident beam of coherent light is divergent;

Fig. 4  shows the device of Fig. 3 having a porous layer arranged above the planar surface;

Fig. 5  shows another embodiment of a device according to the invention having a non-transparent section as beam stop at an opposite outer surface of the substrate, wherein the incident beam of coherent light is parallel;

Fig. 6  shows a side view on the device of Fig. 1 and a variant for the arrangement of the beam generation location and the detection location relative to the device;

Figs. 7a-7b      show a top view on the device of Fig. 1 having arranged thereon the plurality of lines in a predetermined surface area arranged relative to the beam generation location and the detection location to allow for total reflection of the coherent light;

Fig. 8      shows a side view of the device of Fig. 7a;

Figs. 9-12      show steps of a sandwich-assay carried out for the detection of binding affinities; and

Fig. 13      shows a variant of the sandwich-assay of Figs. 9-12 using a different scattering enhancer.

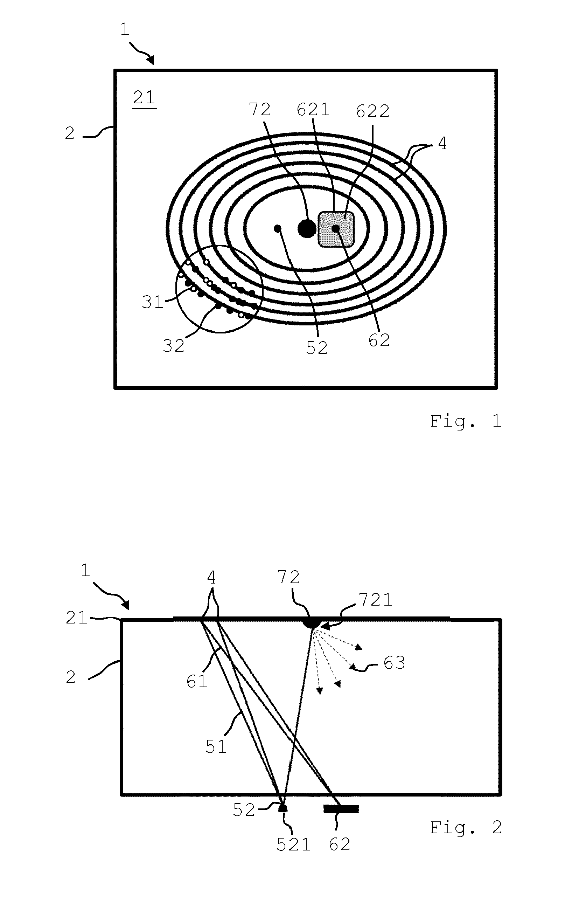

**Fig. 1** shows an embodiment of a device 1 according to the invention. Structurally, one plurality of curved lines 4 is arranged at a planar surface 21 of a transparent substrate 2. In an enlarged view within the circle shown in Fig. 1, binding sites 31 and target molecules 32 are illustrated. Because the density of the curved lines is high, only each fiftieth individual curved line 4 is shown in Fig. 1 together with some adjacent lines. In reality, also the spaces between the groups of curved lines 4 shown in Fig. 1 are filled with curved lines. Curved lines 4 in Fig. 1 are arranged to define ellipses. The elliptical arrangement is such that the integrated optical path length of coherent light 51 from a beam generation location 52 to a specific curved line 4 and the optical path length of a diffracted portion 61 of the coherent light from the specific curved line 4 to a detection location 62 are constant for each line. Curved lines 4 are arranged at an adjacent distance such that the integrated optical path length of coherent light from beam generation location 52 to different curved lines 4 and of the diffracted portion 61 of the coherent light from the respective different curved lines 4 to detection location 62 has a difference which is a multiple integer of the predetermined wavelength of the coherent light 63. In the present example, the distance between adjacent curved lines is in the range of about 300 nm to 600 nm for a wavelength of the coherent light of 635 nm. Beam generation location 52 and a sensing surface (not shown) of CCD or CMOS detector 621 forming detection location 62 are arranged in a common plane parallel to planar surface 21 in a predetermined distance below device 1. A beam stop is arranged inside substrate 2. The beam stop is a deflector body 72 arranged inside substrate 2 at planar surface 21 and is arranged in between beam generation location 52 and detection location 62 along the path of propagation of coherent light propagating therebetween.
The use of the device of Fig. 1 in the detection of binding affinities and the arrangement of curved lines 4 are described in context of **Fig. 2.**
In use, incident beam of coherent light 51 is generated at beam generation location 52 (e. g. a laser as point light source 521 (a spot light source) from which a divergent incident beam of coherent light emanates). Light emanating from beam generation location 52 impinges at curved lines 4 (the lines are not shown as separate lines in the present view). Incident beam 51 is illustrated to propagate to two different curved lines 4 of the plurality of curved lines 4. After being diffracted at binding sites 31 bound to target molecule 32, diffracted portions 61 of coherent light impinge at detection location 62. Impinging diffracted portions 61 of light diffracted at different lines have in detection location 62 a difference in the propagated optical path (beam generation location - respective line - detection location) of a multiple integer of the wavelength of the coherent light and contribute to a maximum intensity.
Curved lines 4 are arranged so that the integrated optical path length of coherent light 51 from beam generation location 52 to different curved lines 4 (not separately shown) and of diffracted portion 61 from respective different curved lines 4 to detection location 62 is a multiple integer of the predetermined wavelength of the coherent light. Hence for different lines 4, the respective integrated optical path length is of a difference which is predetermined to be a multiple integer of the wavelength of the coherent light.
To prevent reflected portions 63 (as non-diffracted portion) of incident beam of coherent light 51 at detection location 62, proximate to planar surface 21, a half sphere (or a polygon) is arranged within substrate 2 as deflector body 72. Deflector body 72 has an outer surface 721 of a convex shape to be capable of scattering off incident coherent light in different directions. This reduces the intensity of light reflected thereon into the direction of the detection location. Hence deflector body 72 allows that, reflected light 63 does not impinge on detection location 62, or is at least greatly reduced. Deflector body 72 is arranged at a position at which it eliminates (or reduces) the reflection of incident beam 51 at the planar surface 21 towards detection location 62.

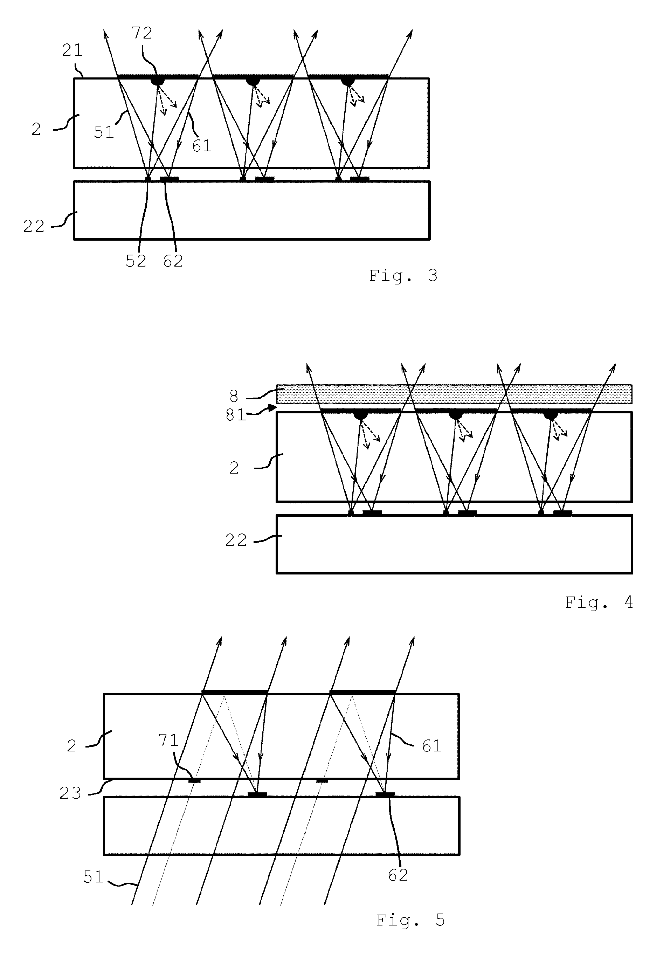

In **Fig 3,** a divergent incident beam of coherent light 51 is generated at beam generation location 52 which is arranged at a carrier 22. Carrier 22 may be a further planar substrate having, for example, arranged thereon a laser light source. The coherent light is diffracted so that diffracted portion 61 impinges at detection location 62 arranged at carrier 22. Beam generation location 52 and detection location 62 are arranged at carrier 22 so as to be arranged in a common plane parallel to planar surface 21.
Another variant is shown in **Fig. 4** which is in principle the device shown in Fig. 3 having arranged thereon a porous layer 8. Porous layer 8 is arranged in a distance to planar surface 21 to form a channel 81 which allows for flowing the binding sites, the target molecule or a complementary binder molecule (not shown) in a fluid therethrough for the application thereof.

In **Fig. 5** another embodiment of a device 1 is shown having a non-transparent section 71 as beam stop. Non-transparent section 71 is arranged at an opposite outer surface 23 of substrate 2. A parallel incident beam of coherent light 51 is diffracted at curved lines (not shown) so that diffracted coherent light 61 impinges at detection location 62. A part of the parallel incident beam of coherent light 51 which will form a reflected portion (as non-diffracted portion) impinging at detection location 62 is masked out by non-transparent section 71.

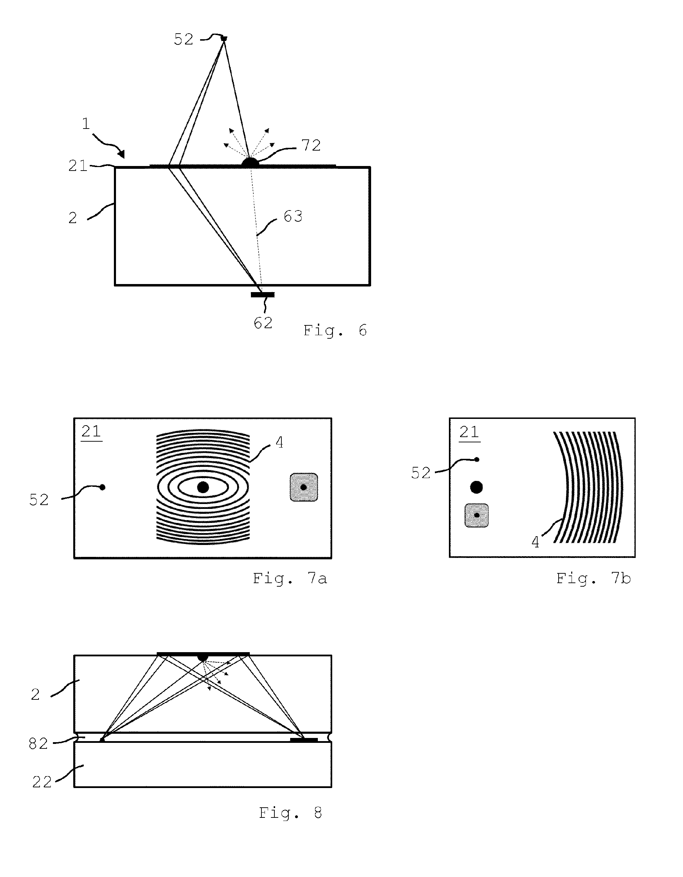

**Fig. 6** is a side view of the device as it has in principle been already explained in reference to Fig. 2. However, in difference thereto, the beam generation location 52 and the detection location 62 are arranged at opposite sides of device 1 (beam generation 52 above and detection location 62 below). In this example, the beam stop is a deflector body 72 arranged at the planar surface 21 so as to prevent non-diffracted portions 63 (refracted portions) of the incident beam of coherent light at the detection location 62. The refracted portion 63 is in the shown example the part of the incident beam of light which changes the direction of propagation so as to propagate into the detection location when passing the interface between the substrate and the medium surrounding the substrate.

Both, **Fig. 7a** and **Fig. 7b,** relate to another aspect according to which the beam generation location is arranged with respect to the plurality of curved lines 4 on the planar surface 21 so that the incident beams of coherent light impinge at plurality of curved lines 4 under an angle of total reflection, in particular "total internal reflection". Hence, no light of the totally reflected incident beam of coherent light propagates out of the substrate through the interface between the substrate and the medium above the planar surface 21. Actually, the totally reflected light penetrates a predetermined distance above the outer surface to be diffracted at the plurality of curved lines 4. For total reflection the predetermined distance is given by the penetration depth of the evanescent field at the point of total internal reflection.

Furthermore, Fig. 7a and Fig. 7b, relate to another aspect according to which the detection location 62 is arranged with respect to plurality of curved lines 4 on the planar surface 21 so that the diffracted beams of coherent light emanate (i.e. interfere outgoing) from the plurality of curved lines 4 under an angle to the normal of the planar surface 21 which is larger than the critical angle of total internal reflection; the latter angle depends on the refractive indices of the substrate and the medium above the substrate. Hence, no light from above the planar surface 21 reaches the detection location through the region covering the plurality of curved lines 4 on the planar surface 21. This aspect of the invention increases the detection sensitivity of the device in use.

As can be seen in **Fig. 8,** which is the side view of the device of which in Fig. 7a the planar surface is shown, total reflection of the incident beam of coherent light is achieved by arranging the beam generation location relative to the plurality of adjacently arranged curved lines so that the incident light impinges under the angle for which total reflection occurs (an angle larger than a particular critical angle with respect to the normal to the surface, which critical angle depends on the refractive indices of the substrate and the medium above the substrate). In between of substrate 2 and carrier 22, an immersion liquid 82 of a predetermined refractive index (e.g. the same as of the substrate and/or the carrier) is provided. As in Fig. 8, diffracted beams of coherent light emanate from the plurality of curved lines 4 under an angle to the normal of the planar surface 21 which is larger than the critical angle of total (internal) reflection.

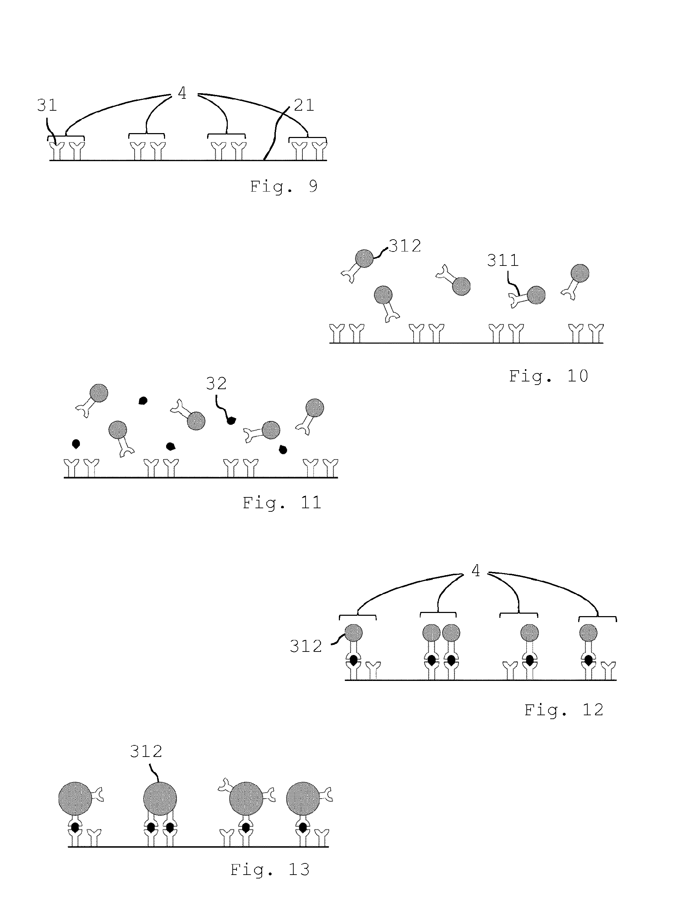

Figs. 9-12 illustrate four steps of a sandwich-assay carried out on a planar surface 21 of a device according to the invention. Such a "sandwich" comprises a binding site 31 bound to planar surface 21, target molecule 32 bound to binding site 31 and a complementary binder molecule 311 bound to target molecules 32. In a first step **(Fig. 9)** binding sites 31 are arranged on planar surface 21 of such a device along a plurality of curved lines 4.

Complementary binder molecule 311 shown in **Fig. 10** has a scattering enhancer 312 which is in a particular example a gold-nano particle capable of increasing intensity of diffracted portion 61 of coherent light diffracted thereon. Complementary binder molecules 311 are randomly disposed in the proximity of the binding sites, e.g. by spraying them onto planar surface 21 or attaching them to the porous layer (see Fig. 4, 8). Randomly disposed scattering enhancers 312 provide no interference maximum in the detection location but only scattered background light.

Target molecules 32 are applied to binding sites 31 **(Fig. 11)** so that at such binding site 31, target molecule 32 and complementary binder molecule 311 having scattering enhancer 312 to form a "sandwich". Hence, scattering enhancer 312 is arranged along plurality of curved lines 4 **(Fig. 12).** The signal provided by light diffracted by scattering enhancers 312 arranged along plurality of curved lines 4 is of a high intensity compared with the signal provided by the remaining randomly arranged scattering enhancers 312.

Illustrated in **Fig. 13** is a variant of the sandwich-assay shown in Figs. 9-12. According to a first aspect, the scattering enhancer 321 provided at the complementary binder molecules is larger in size (increased size compared to Figs. 9-12) so as to provide a higher intensity of the diffracted portion to increase sensitivity of the device in use. According to a second aspect, the scattering enhancer 321 binds to more than one (e.g. two, three, etc.) complementary binder molecules. Two complementary binder molecules allow for binding to two different target molecules simultaneously or subsequently (within short periods of time) to allow for a multiple bond interaction having a combined strength (avidity).

# EP 2 827 130 A1

**Claims**

1. Device (1) for use in the detection of binding affinities, the device (1) comprising a substrate (2) with a planar surface (21) having arranged thereon a plurality of binding sites (31) capable of binding with a target molecule (32), wherein the binding sites (31) are arranged on the planar surface (21) along a plurality of adjacently arranged curved lines (4) which are spaced from one another by a distance to cause an incident beam of coherent light (51) of a predetermined wavelength generated at a beam generation location (52) to be diffracted at the binding sites (31) with the bound target molecule (32) in a manner such that diffracted portions (61) of the incident beam of coherent light (51) interfere at a detection location (62) with a difference in optical path length which is a multiple integer of the predetermined wavelength of the coherent light to provide a signal representative of the binding affinity of the binding sites (31) and the target molecule (32), and further comprising a beam stop (7) which is arranged to prevent non-diffracted portions (63) of the incident beam of coherent light (51) at the detection location (62).

2. Device (1) according to claim 1, wherein the substrate (2) comprises a material which is transparent to light of the predetermined wavelength of the coherent light so as to allow for propagation of the incident beam of coherent light (51) and of such a diffracted portion (61) of coherent light through the substrate (2).

3. Device (1) according to claim 2, wherein the beam stop (7) comprises an non-transparent section (71) which is arranged on or within the substrate (2) in a manner to prevent non-diffracted portions (63) of the incident beam of coherent light (51) at the detection location (62).

4. Device (1) according to claim 2, wherein the beam stop (7) comprises an anti-reflective section arranged at the planar surface (21) of the substrate (2).

5. Device (1) according to claim 2, wherein the beam stop (7) comprises a deflector body (72) arranged on or within the substrate (2), the deflector body (72) having a curved outer surface (721) which is capable of scattering off the coherent light in a manner such that the non-diffracted portion (63) of the incident beam of coherent light impinging at the detection location (62) is dissipated.

6. Device (1) according to any one of the preceding claims, wherein the beam generation location (52) and the detection location (62) are arranged on an opposite outer surface (23) of the substrate (2) opposite to the planar surface (21), wherein the beam generation location (52) and the detection location (62) are arranged on the opposite outer surface (23) in a common plane parallel to the planar surface (21).

7. Device (1) according to any one of claims 1 to 5, further comprising a carrier (22) having the beam generation location (52) and the detection location (62) arranged thereon, the carrier (22) being arranged with respect to the substrate (2) such that the beam generation location (52) and the detection location (62) are arranged in a common plane parallel to the planar surface (21).

8. Device (1) according to claim 6 or 7, wherein each line of the plurality of curved lines (4) is arranged on the planar surface (21) such that the integrated optical path length of the coherent light (51) from the beam generation location (52) to the curved line (4) and of the diffracted portion (61) of the coherent light from the respective curved line (4) to the detection location (62) is constant.

9. Device (1) according to claim 8, wherein adjacent curved lines (4) of the plurality of curved lines (4) are arranged at a distance such that the integrated optical path length of the coherent light (51) from the beam generation location (52) to different curved lines (4) and of the diffracted portions (61) of the coherent light from the respective different curved lines (4) to the detection location (62) has a difference which is a multiple integer of the predetermined wavelength of the coherent light.

10. Device (1) according to any one of the preceding claims, wherein the detection location (62) is a sensing surface (622) of a CCD or CMOS detector (621) having a plurality of pixels arranged on the sensing surface (622) in a grid-like manner, each pixel having a size so as to be capable of detecting less than half of the complete spatially distributed intensity of the interfering diffracted portions (61) at the detection location (62) in one single pixel.

11. Device (1) according to any one of the preceding claims, wherein the beam generation location (52) is a point light source (521) capable of generating a divergent incident beam of coherent light (51).

**12.** Device (1) according to any one of the preceding claims, further comprising an outer layer (8) arranged at the planar surface (21).

**13.** Method for detecting binding affinities comprising the steps of:

- providing a device (1) according to any one of the preceding claims;
- applying a plurality of target molecules (32) to the binding sites (31);
- at a beam generation location (52) generating an incident beam of coherent light (51) to be diffracted at the binding sites (31) with the target molecule (32) bound thereto in a manner such that diffracted portions (61) of the incident beam of coherent light (51) interfere at a detection location (62) with a difference in optical path length which is a multiple integer of the predetermined wavelength of the incident beam of coherent light (51) to provide a signal representative of the binding sites (31) with the target molecule (32) bound thereto at the detection location (62);
- at the detection location (62) measuring the signal representative of the binding sites (31) with the target molecule (32) bound thereto; and
- detecting the binding affinity of the binding sites (31) to the target molecule (32) by comparing the signal representative of the binding sites (31) with the target molecule (32) bound thereto with a reference signal representative of the binding sites (31) only.

**14.** Method according to claim 13, the method before applying the plurality of target molecules (32) to the binding sites (31) comprising the steps of:

- at the beam generation location (52) generating the incident beam of coherent light (51) which is to be diffracted at the binding sites (31) only in a manner such that diffracted portions (61) of the incident beam of coherent light (51) interfere at the detection location (62) with a difference in optical path length which is a multiple integer of the predetermined wavelength of the incident beam of coherent light (51) to provide a signal representative of the binding sites (31) only at the detection location (62); and
- at the detection location (62) measuring the signal representative of the binding sites (31) only to provide the reference signal.

**15.** Method according to claim 13 or 14, further comprising the step of:

- applying a plurality of complementary binder molecules (311) to the binding sites (31), the complementary binder molecules (311) being capable of binding to the target molecules (32) bound to the binding sites (31), wherein the complementary binder molecules (311) comprise a scattering enhancer (312).

Fig. 1

Fig. 2

Fig. 3

Fig. 4

Fig. 5

Fig. 6

Fig. 7a

Fig. 7b

Fig. 8

Fig. 9

Fig. 10

Fig. 11

Fig. 12

Fig. 13

Europäisches
Patentamt

European
Patent Office

Office européen
des brevets

**EUROPEAN SEARCH REPORT**

Application Number

EP 13 17 6536

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 483 096 B1 (KUNZ RINO E [CH] ET AL) 19 November 2002 (2002-11-19) * paragraph [0066]; figures 11-15 * ----- | 1-15 | INV. G01N21/64 G01N33/543 G01N33/551 |
| A | US 4 647 544 A (NICOLI DAVID F [US] ET AL) 3 March 1987 (1987-03-03) * figures 1,3 * ----- | 1-15 | |
| A | US 2002/025534 A1 (GOH M CYNTHIA [CA] ET AL) 28 February 2002 (2002-02-28) * figures 1a-1b * ----- | 1-15 | |
| A | US 4 931 384 A (LAYTON DEREK G [GB] ET AL) 5 June 1990 (1990-06-05) * figure 3 * ----- | 1-15 | |
| A | US 4 882 288 A (NORTH JOHN R [GB] ET AL) 21 November 1989 (1989-11-21) * figures 2a,2b,3 * ----- | 1-15 | |
| A | US 4 876 208 A (GUSTAFSON ERIC K [US] ET AL) 24 October 1989 (1989-10-24) * figures 1, 4 * ----- | 1-15 | TECHNICAL FIELDS SEARCHED (IPC) G01N |
| A | US 5 124 172 A (BURRELL ROBERT E [CA] ET AL) 23 June 1992 (1992-06-23) * figures 4-5 * ----- | 1-15 | |

The present search report has been drawn up for all claims

| Place of search | Date of completion of the search | Examiner |
|---|---|---|
| Munich | 26 November 2013 | Mason, William |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 13 17 6536

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2013

| Patent document cited in search report | | Publication date | Patent family member(s) | | Publication date |
|---|---|---|---|---|---|
| US 6483096 | B1 | 19-11-2002 | AT | 244883 T | 15-07-2003 |
| | | | DE | 69909480 D1 | 14-08-2003 |
| | | | DE | 69909480 T2 | 15-04-2004 |
| | | | EP | 1085315 A1 | 21-03-2001 |
| | | | US | 6483096 B1 | 19-11-2002 |
| US 4647544 | A | 03-03-1987 | DE | 3584742 D1 | 09-01-1992 |
| | | | EP | 0167335 A2 | 08-01-1986 |
| | | | US | 4647544 A | 03-03-1987 |
| US 2002025534 | A1 | 28-02-2002 | AT | 486275 T | 15-11-2010 |
| | | | AU | 4217301 A | 03-10-2001 |
| | | | CA | 2403427 A1 | 27-09-2001 |
| | | | EP | 1266207 A2 | 18-12-2002 |
| | | | JP | 4999249 B2 | 15-08-2012 |
| | | | JP | 2003528311 A | 24-09-2003 |
| | | | US | 2002025534 A1 | 28-02-2002 |
| | | | US | 2006099649 A1 | 11-05-2006 |
| | | | WO | 0171322 A2 | 27-09-2001 |
| US 4931384 | A | 05-06-1990 | AU | 570425 B2 | 17-03-1988 |
| | | | CA | 1237645 A1 | 07-06-1988 |
| | | | DE | 3376685 D1 | 23-06-1988 |
| | | | EP | 0112721 A2 | 04-07-1984 |
| | | | JP | 2502222 B2 | 29-05-1996 |
| | | | JP | H0627742 B2 | 13-04-1994 |
| | | | JP | H0650972 A | 25-02-1994 |
| | | | JP | S60500309 A | 07-03-1985 |
| | | | US | 4931384 A | 05-06-1990 |
| | | | US | 5118608 A | 02-06-1992 |
| | | | WO | 8402578 A1 | 05-07-1984 |
| US 4882288 | A | 21-11-1989 | AT | 62074 T | 15-04-1991 |
| | | | AU | 591070 B2 | 30-11-1989 |
| | | | AU | 4806285 A | 08-04-1986 |
| | | | CA | 1263599 A1 | 05-12-1989 |
| | | | DE | 3582296 D1 | 02-05-1991 |
| | | | EP | 0178083 A1 | 16-04-1986 |
| | | | JP | H0658370 B2 | 03-08-1994 |
| | | | JP | S62500736 A | 26-03-1987 |
| | | | NO | 168273 B | 21-10-1991 |
| | | | US | 4882288 A | 21-11-1989 |
| | | | WO | 8601901 A1 | 27-03-1986 |
| US 4876208 | A | 24-10-1989 | NONE | | |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 13 17 6536

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2013

| Patent document cited in search report | | Publication date | Patent family member(s) | | Publication date |
|---|---|---|---|---|---|
| US 5124172 | A | 23-06-1992 | AT | 143731 T | 15-10-1996 |
| | | | CA | 1337173 C | 03-10-1995 |
| | | | DE | 69028728 D1 | 07-11-1996 |
| | | | DE | 69028728 T2 | 06-03-1997 |
| | | | DK | 0395300 T3 | 02-12-1996 |
| | | | EP | 0395300 A2 | 31-10-1990 |
| | | | HK | 1006039 A1 | 05-02-1999 |
| | | | IN | 178593 A1 | 24-05-1997 |
| | | | JP | 2993513 B2 | 20-12-1999 |
| | | | JP | H02300661 A | 12-12-1990 |
| | | | US | 5124172 A | 23-06-1992 |

EPO FORM P0459

For more details about this annex : see Official Journal of the European Patent Office, No. 12/82